# EUROPEAN PATENT APPLICATION

(11) **EP 0 897 702 A2**
(43) Date of publication of application: **24.02.1999**
(21) Application number: 98305081.6
(22) Date of filing: 26.06.1998
(51) Int. Cl.: A61F 2/16

(54) **Intraocular lens implant**

(30) Priority: 26.06.1997 US 882973
(71) Applicant: Visioncare Ltd., Yehud 56101 (IL)
(72) Inventor: Gross, Yosef, 73160 (IL); Lipshitz, Issac, Herzliya Pituach 36448 (IL); Gedeon, Dotan, Yehud 56275 (IL)
(74) Representative: Gold, Tibor Z.

(57) **Abstract**

An intraocular lens implant 10 for implantation in the interior of a human eye 12 has an anterior side 14 and a posterior side 16. The implant 10 comprises a telescope 18 which extends through at least a portion of a lens capsule 21 of the eye 12 and forwardly thereof toward the anterior side 14 of the eye, the telescope not penetrating the vitreous of the eye.

## Description

### FIELD OF THE INVENTION

The present invention relates to intraocular lens implants generally.

### BACKGROUND OF THE INVENTION

Various types of intraocular lens implants are known in the patent literature. Particular reference is made to U.S. Patents 5,391,202 and 5,354,335 of the present applicant/assignee and to the references cited therein. Other relevant references include European Published Patent Application EP-A-212616, U.S. Patents 4,074,368; 4,172,297; 4,759,761 and 5,275,623 and French Published Patent Application 2,666,735.

The utility of intraocular lens implants is described in the above patent references. The disclosures of the above-mentioned publications are hereby incorporated by reference.

### SUMMARY OF THE INVENTION

The present invention seeks to provide improved intraocular lens implants.

There is thus provided in accordance with a preferred embodiment of the present invention an intraocular lens implant for implantation in the interior of a human eye having an anterior side and a posterior side, the implant comprising a telescope which extends through at least a portion of a lens capsule of the eye and forwardly thereof toward the anterior side of the eye, the telescope not penetrating the vitreous of the eye.

There is additionally provided in accordance with a preferred embodiment of the present invention an intraocular lens implant for implantation in the interior of a human eye having an anterior side and a posterior side and a lens capsule, the implant comprising a telescope which extends through at least a portion of a lens capsule of the eye and forwardly thereof toward the anterior side of the eye, the telescope not penetrating the vitreous of the eye.

In accordance with a preferred embodiment of the present invention, the intraocular lens implant is supported within the lens capsule by loops, in the absence of a lens within the lens capsule.

In accordance with a preferred embodiment of the present invention, the telescope is tilted such that light from outside the eye is focused by the telescope on a low resolution but operative section of the retina.

In accordance with a preferred embodiment of the present invention, the telescope may include one or more lenses having a graded index of refraction. Alternatively, one or more lenses in the telescope may be holographic. Additionally in accordance with a preferred embodiment of the present invention the telescope may employ doublet lenses to avoid chromatic aberrations. Alternatively or additionally, additional lenses may be added intermediate lenses at ends of the telescope for this purpose.

There is additionally provided in accordance with a preferred embodiment of the present invention a method for manufacturing an intraocular insert telescope including providing a telescope body and at least two lenses and joining the at least two lenses to the telescope body in a manner which substantially avoids optical distortions. In accordance with a preferred embodiment of the invention, the method uses laser fusing to join the lenses to the telescope body. Alternatively or additionally, the method employs glass particles having a low temperature melting point as a joining medium.

There is additionally provided in accordance with a preferred embodiment of the present invention a telescope having a telescope body and at least two lenses joined in a manner which substantially avoids optical distortions. In accordance with a preferred embodiment of the invention, the lenses are joined to the telescope body by laser fusing. Alternatively or additionally, glass particles having a low temperature melting point are employed as a joining medium.

### BRIEF DESCRIPTION OF THE DRAWINGS

The present invention will be understood and appreciated more fully from the following detailed description, taken in conjunction with the drawings in which:
Fig. 1 is a simplified pictorial illustration of an intraocular inserted constructed and operative in accordance with a preferred embodiment of the present invention located within a human eye;
Fig. 2 is a simplified pictorial illustration of an intraocular inserted constructed and operative in accordance with another preferred embodiment of the present invention located within a human eye;
Fig. 3 is a simplified pictorial illustration of an intraocular inserted constructed and operative in accordance with still another preferred embodiment of the present invention located within a human eye;
Fig. 4 is a simplified pictorial illustration of an intraocular inserted constructed and operative in accordance with yet another preferred embodiment of the present invention located within a human eye;
Fig. 5 is a simplified pictorial illustration of an intraocular inserted constructed and operative in accordance with a further preferred embodiment of the present invention located within a human eye; and
Fig. 6 is a simplified illustration of a technique for manufacture of an intraocular insert telescope in accordance with a preferred embodiment of the present invention located within a human eye.

### DETAILED DESCRIPTION OF PREFERRED EMBODIMENTS

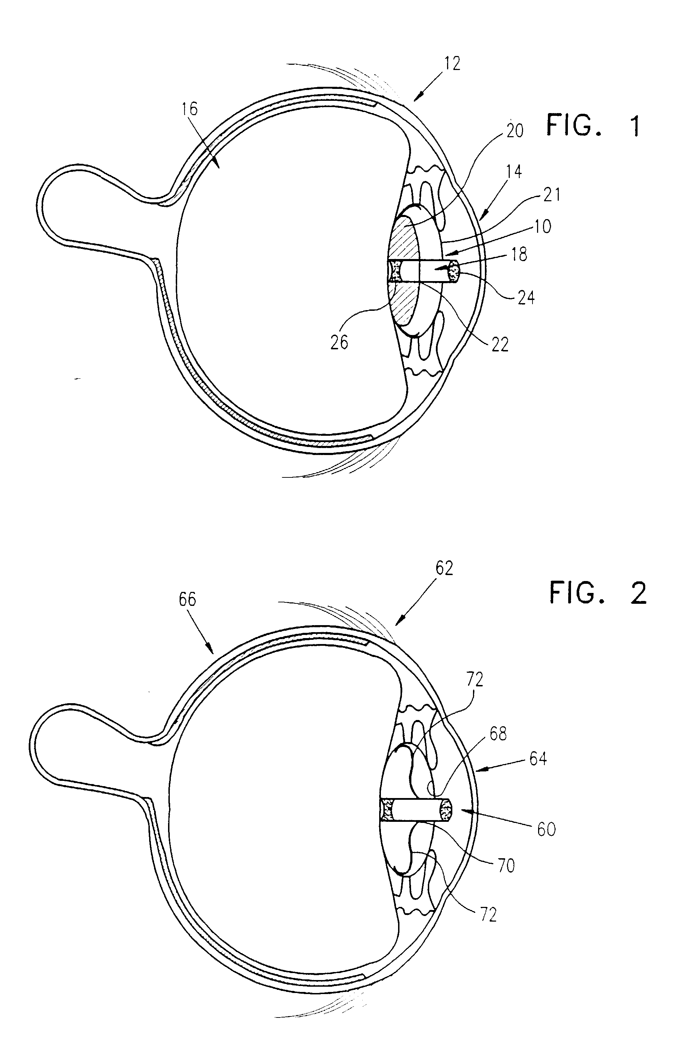

Reference is now made to Fig. 1, which is a simplified pictorial illustration of an intraocular insert constructed and operative in accordance with a preferred embodiment of the present invention located within a human eye.

As seen in Fig. 1, there is provided an intraocular lens implant, indicated generally by reference numeral 10, which is implanted in the interior of a human eye 12 having an anterior side 14 and a posterior side 16. In the illustrated embodiment, the implant comprises a telescope 18 which extends through at least a portion of a carrying lens 20, which is preferably implanted in a lens capsule 21 of the eye 12. The telescope 18 preferably extends through at least a portion of the lens capsule 21 and forwardly thereof toward the anterior side 14 of the eye. It is a particular feature of the invention that the telescope does not penetrate the vitreous of the eye.

In the illustrated embodiment of Fig. 1, it is seen that the telescope comprises a telescope body 22, typically of circular cylindrical configuration and formed of glass or other suitable bio-compatible non-porous materials. Alternatively other materials may be used when coated with suitable bio-compatible non-porous materials. Sealed to the telescope body 22 are preferably provided forward and rearward lenses, respectively designated by reference numerals 24 and 26.

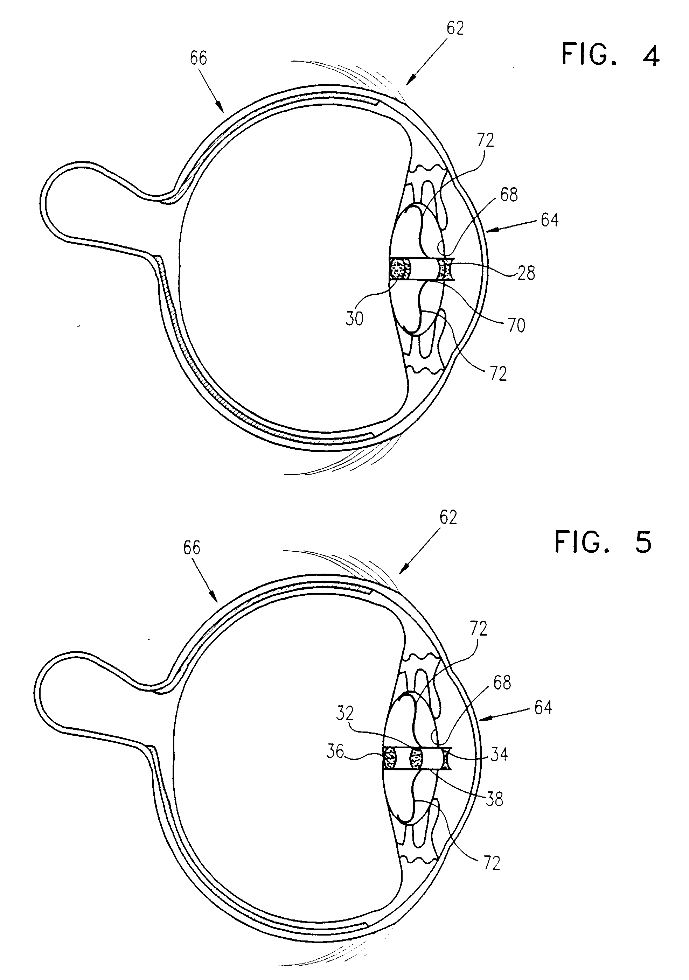

According to an alternative embodiment of the present invention, illustrated in Fig. 4, doublet lens 28 and 30 may be employed to avoid chromatic aberrations.

According to another alternative embodiment of the invention, illustrated in Fig. 5, an additional lens 32 may be provided spaced from forward and rearward lenses 34 and 36 respectively along a telescope body 38. This structure may help to prevent or reduce chromatic aberrations.

In accordance with a preferred embodiment of the present invention a method for manufacturing an intraocular insert telescope is provided, as illustrated in Fig. 6.

The method includes providing a telescope body 50 and at least two lenses 52 and 54 and sealing joining the lenses 52 and 54 to the telescope body 50 in a manner which substantially avoids optical distortions. In accordance with one embodiment of the invention, the method uses laser fusing to join the lenses to the telescope body. Alternatively or additionally, the method employs glass particles 55 having a low temperature melting point as a joining medium.

There is thus realized in accordance with a preferred embodiment of the present invention a telescope having a telescope body and at least two lenses joined in a manner which substantially avoids optical distortions. In accordance with a preferred embodiment of the invention, the lenses are joined to the telescope body by laser fusing. Alternatively or additionally, glass particles having a low temperature melting point are employed as a joining medium.

Referring now to Fig. 2, there is seen an intraocular lens implant 60 constructed and operative in accordance with an alternative embodiment of the present invention. The implant 60 is shown in Fig. 2 implanted in the interior of a human eye 62 having an anterior side 64 and a posterior side 66 and a lens capsule 68. The implant comprises a telescope 70 which extends through at least a portion of a lens capsule 68 of the eye and forwardly thereof toward the anterior side of the eye, the telescope not penetrating the vitreous of the eye. The telescope may be constructed as described hereinabove with reference to any of Figs. 1, 4, 5 and 6.

In accordance with a preferred embodiment of the present invention, the intraocular lens implant is supported within the lens capsule by loops 72, in the absence of a lens within the lens capsule.

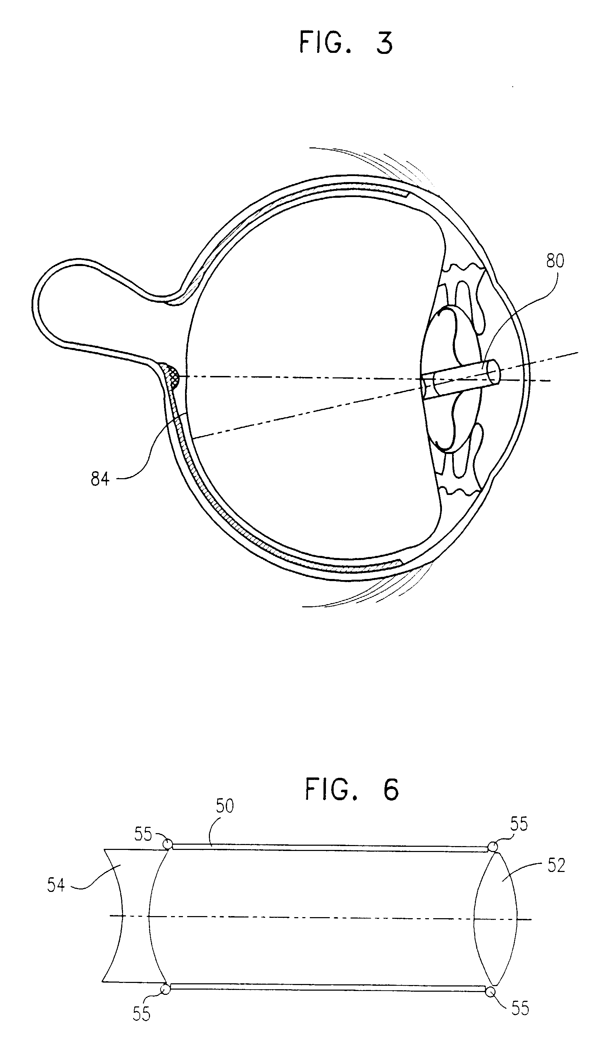

Referring now to Fig. 3, it is seen that in accordance with a preferred embodiment of the present invention, the telescope of Fig. 2, here indicated by reference numeral 80 may be tilted such that light from outside the eye is focused by the telescope on a low resolution but operative section of the retina, indicated by reference numeral 84. In this embodiment, the telescope 80 need not provide a wide field of view.

It is appreciated that in accordance with the present invention, the telescopes described hereinabove may include one or more lenses having a graded index of refraction. Alternatively, one or more lenses in the telescope may be holographic. Additionally in accordance with a preferred embodiment of the present invention the telescope may employ doublet lenses to avoid chromatic aberrations. Alternatively or additionally, additional lenses may be added intermediate lenses at ends of the telescope for this purpose.

It will be appreciated by persons skilled in the art that the present invention is not limited by what has been particularly shown and described hereinabove. Rather the scope of the present invention includes both combinations and subcombinations of the various features described hereinabove as well as variations and further developments thereof which would occur to persons skilled in the art upon reading the above description and which are not in the prior art.

## Claims

1. An intraocular lens implant for implantation in the interior of a human eye having an anterior side and a posterior side, the implant comprising a telescope which extends through at least a portion of a lens capsule of the eye and forwardly thereof toward the anterior side of the eye, the telescope not penetrating the vitreous of the eye.

2. An intraocular lens implant according to claim 1 and wherein said telescope is supported within the lens capsule by a carrying lens; or by loops, in the absence of a lens within the lens capsule.

3. An intraocular lens implant according to claim 1 or 2 and wherein said telescope is tilted such that light from outside the eye is focused by the telescope on a low resolution but operative section of the retina.

4. An intraocular lens implant according to any preceding claim and wherein the telescope includes lenses having a graded index of refraction.

5. An intraocular lens implant according to any preceding claim and wherein said telescope includes at least one holographic lens; or at least one doublet lens; or at least three mutually spaced lenses.

6. A method of manufacturing an intraocular insert telescope including providing a telescope body and at least two lenses and sealingly joining the at least two lenses to the telescope body in a manner which substantially avoids optical distortions.

7. An intraocular telescope having a telescope body and at least two lenses joined in a manner which substantially avoids optical distortions.

8. A method according to claim 6 or a telescope according to claim 7 and employing laser fusing to sealingly join the lenses to the telescope body.

9. A method according to claim 6 or 8 and employing glass particles having a low temperature melting point as a joining medium.

10. An intraocular telescope according to claim 7 or 8 and wherein glass particles having a low temperature melting point are employed as a joining medium.
